(19) Europäisches Patentamt
European Patent Office
Office européen des brevets

(11) EP 4 424 334 A1

(12) **EUROPEAN PATENT APPLICATION**
published in accordance with Art. 153(4) EPC

(43) Date of publication:
**04.09.2024 Bulletin 2024/36**

(21) Application number: **22886870.9**

(22) Date of filing: **21.10.2022**

(51) International Patent Classification (IPC):
***A61L 29/08*** (2006.01) ***A61L 27/16*** (2006.01)
***A61L 27/52*** (2006.01) ***A61L 29/14*** (2006.01)
***A61M 25/00*** (2006.01) ***A61M 25/09*** (2006.01)

(52) Cooperative Patent Classification (CPC):
**A61L 27/16; A61L 27/52; A61L 29/08; A61L 29/14; A61M 25/00; A61M 25/09**

(86) International application number:
**PCT/JP2022/039207**

(87) International publication number:
**WO 2023/074543 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.10.2021 JP 2021177323**

(71) Applicant: **ASAHI INTECC CO., LTD.**
**Seto-shi, Aichi, 489-0071 (JP)**

(72) Inventors:
- **FUTAMI, Soichi**
  **Seto-shi, Aichi 489-0071 (JP)**
- **TAKATA, Yumi**
  **Seto-shi, Aichi 489-0071 (JP)**
- **YAMAMOTO, Shinpei**
  **Hakusan-shi, Ishikawa 924-0057 (JP)**

(74) Representative: **Prinz & Partner mbB**
**Patent- und Rechtsanwälte**
**Rundfunkplatz 2**
**80335 München (DE)**

# (54) ELONGATED MEDICAL INSTRUMENT AND METHOD FOR PRODUCING SAME

(57) Provided is an elongated medical instrument on which a hydrogel film is formed using a simpler method. In the elongated medical instrument including a base material and a swollen gel film coating the base material, the swollen gel film includes a polymer (a1) including a polymer unit with a betaine structure and a polymer unit with a carboxyl group (excluding the polymer unit with the betaine structure).

FIG.1

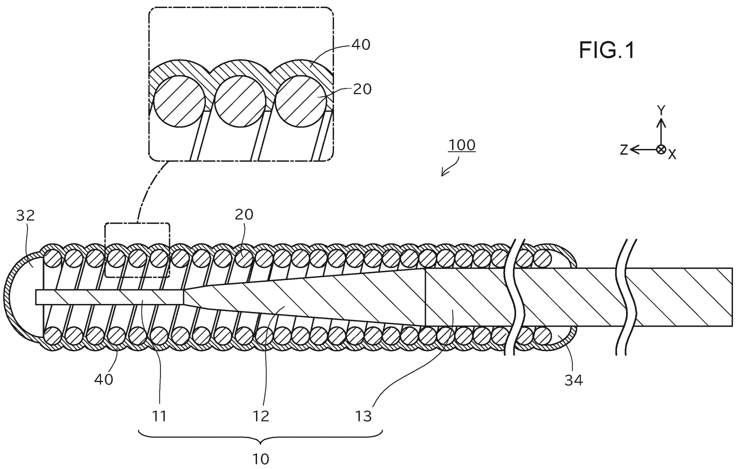




Processed by Luminess, 75001 PARIS (FR)

## Description

TECHNICAL FIELD

The technology disclosed herein relates to an elongated medical instrument and a method for manufacturing the same.

BACKGROUND ART

In general, a medical instrument or the like inserted into a body, such as into a blood vessel, requires good insertability into the blood vessel or the like and good operability in the blood vessel or the like. Thus, for example, an elongated medical instrument such as a guidewire or a catheter is coated, on its surface, with a hydrophilic polymer or the like to provide good lubricity to the guidewire surface, thereby ensuring good insertability and operability.

On the other hand, as a polymer composition coated on the elongated medical instrument, there is known a surface-modifying additive composition including an oligomeric or polymeric additive formed of two or more of a zwitterionic monomer or a polyalkylene glycol monomer, a silicone or fluorocarbon monomer, or a combination thereof, or an alkyl substituted methacrylate, acrylate, acrylamide, or vinyl monomer, or a combination thereof. The above-mentioned coating is performed by applying the surface-modifying additive composition onto the medical instrument by dip coating or the like. Further, a carboxybetaine monomer is disclosed as a type of the above-mentioned zwitterionic monomer (see, e.g., Patent literature 1).

CITATION LIST

Patent Literature

Patent Literature 1: JP 2018-524029 W

SUMMARY OF INVENTION

TECHNICAL PROBLEM

From the viewpoint of ensuring lubricity on the surface of the elongated medical instrument or the like, a highly biocompatible hydrogel film may be formed on the surface. However, for forming the hydrogel film, a method involving complicated steps is conventionally adopted. Examples of such a method include a method in which a crosslinking agent or the like is added to a hydrophilic monomer to produce a crosslinked hydrophilic polymer, and a method in which a hydrophilic polymer is applied and then UV irradiated to produce a crosslinked hydrophilic polymer. Further, according to the above-mentioned method using the crosslinking agent or the like, for example, a step of removing the unreacted crosslinking agent may be required for maintaining the performance such as biocompatibility. Thus, it has been desired to provide a method for forming a hydrogel film on the surface of an elongated medical instrument or the like using a simpler method without going through these complicated steps, and an elongated medical instrument on which the hydrogel film is formed.

Note that the object of providing the method for forming the hydrogel film on the surface of the elongated medical instrument by a simpler method and the elongated medical instrument on which the hydrogel film is formed is not limited to the medical instrument inserted into the body such as the blood vessel, but is common to medical instruments and other base materials that require lubricity.

The present specification discloses a technique that can solve the above-mentioned problems.

SOLUTION TO PROBLEM

The technology disclosed herein can be achieved, for example, in the following forms.

(1) An elongated medical instrument disclosed herein includes a base material and a swollen gel film coating the base material. The swollen gel film includes a polymer (a1) including a repeating unit with a betaine structure and a repeating unit with a carboxyl group (excluding the repeating unit with the betaine structure). According to the elongated medical instrument, it is possible to form the swollen gel film with good lubricity simply by heating without using a crosslinking agent.

(2) In the above-mentioned elongated medical instrument, the repeating unit with the betaine structure may include a repeating unit with an ester-bonded betaine structure.

(3) In the above-mentioned elongated medical instrument, the swollen gel film may have a swelling degree of 180% or more and 900% or less.

(4) In the above-mentioned elongated medical instrument, the swollen gel film may have a molar ratio of the repeating unit with the betaine structure relative to the repeating unit with the carboxyl group in the polymer (a1) of 80:20 to 60:40.

(5) In the above-mentioned elongated medical instrument, the base material may be a guidewire or a catheter.

(6) A method for producing an elongated medical instrument disclosed herein includes an application step of applying a coating agent onto a base material, and a formation step of forming a swollen gel film by heating the coating agent. The coating agent includes a polymer having a repeating unit with an ester-bonded betaine structure, an organic solvent having a dispersion term $\delta D$ of 10 to 24 $MPa^{1/2}$, a polar term $\delta P$ of 5 to 19 $MPa^{1/2}$, and a hydrogen bond term $\delta H$ of 3 to 17 $MPa^{1/2}$ in the Hansen solubility parameters at 25 °C, and having a boiling point of higher than 100 °C, and water. In the formation step, the swollen gel film is formed by hydrolyzing ester bonds in the polymer. According to the method for producing the elongated medical instrument, the coating agent includes the polymer including the repeating unit with the ester-bonded betaine structure and the organic solvent that satisfies the above-mentioned Hansen solubility parameters and has a boiling point of higher than 100°C, making it possible to favorably form the swollen gel film on the base material without going through complicated steps.

(7) In the above-mentioned method for producing the elongated medical instrument, the organic solvent may be at least one selected from N-methyl-2-pyrrolidone (NMP), dimethyl sulfoxide (DMSO), N,N-dimethylformamide (DMF), dimethylacetamide (DMA), diacetone alcohol (DAOH), and diethylene glycol monoethyl ether (EDG).

(8) In the above-mentioned method for producing the elongated medical instrument, the base material may be a guidewire or a catheter, and, in the formation step, a hydrolysis rate of the ester bonds in the polymer may be 20% or more and 40% or less. According to the present method for forming the swollen gel film, a swelling degree can be appropriately adjusted, making it possible to form the swollen gel film with lubricity suitable for the guidewire or the catheter.

BRIEF DESCRIPTION OF DRAWINGS

Fig. 1 is an explanatory diagram schematically showing a configuration of a longitudinal section (YZ section) of a guidewire 100 of a present embodiment.

Figs. 2A, B, C, and D are explanatory diagrams conceptually showing a method for forming a swollen gel film GM of the present embodiment.

Fig. 3 is a reaction formula showing a hydrolysis reaction in the method for forming the swollen gel film GM of the present embodiment.

EMBODIMENTS OF THE INVENTION

A. Embodiment:

A-1. Coating agent CA:

A coating agent CA of the present embodiment includes a polymer PA represented by the following general formula (1) and an organic solvent HS.

<Polymer PA>

As represented by the following formula (1), the polymer PA of the present embodiment includes a repeating unit with an ester-bonded betaine structure (hereinafter referred to as a "structural unit a1").

[Chemical 1]

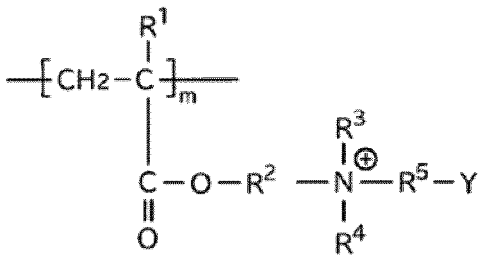

...(1)

a1

(In formula (1), R' is a hydrogen atom or a methyl group, $R^2$ is a linear or branched alkylene group having 1 or more to 6 or less carbon atoms, $R^3$ and $R^4$ are each independently an alkyl group having 1 or more to 4 or less carbon atoms, $R^5$ is a linear or branched alkylene group having 1 or more to 4 or less carbon atoms, Y is -COO- or $-SO_3^-$, and m is an integer of 1 or more)

A proportion of the structural unit a1 in the polymer PA is, for example, 10 to 100 mol%.

Examples of the above-mentioned repeating unit with the ester-bonded betaine structure include a repeating unit derived from N-methacryloyl oxyethyl-N,N-dimethyl ammonium-α-N-methyl carboxy betaine (GLBT), 3-[2-(methacryloyloxy)ethyl]dimethyl ammonio]propionate (CEBMA), or 3-[2-(methacryloyloxy)ethyl]dimethyl ammonio]propane-1-sulfonate (SPBMA). Of these, a repeating unit derived from N-methacryloyl oxyethyl-N,N-dimethyl ammonium-α-N-methyl carboxy betaine (GLBT) is preferable.

As represented by the following general formula (2), in addition to the structural unit a1 represented by the above-mentioned formula (1), the polymer PA may include a repeating unit derived from (meth)acrylic acid hydroxyalkyl ester (hereinafter referred to as a "structural unit a2").

[Chemical 2]

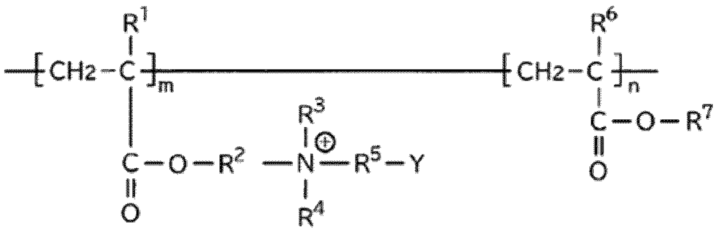

...(2)

a1 a2

(In formula (2), R' is a hydrogen atom or a methyl group, $R^2$ is a linear or branched alkylene group having 1 or more to 6 or less carbon atoms, $R^3$ and $R^4$ each independently an alkyl group having 1 or more to 4 or less carbon atoms, $R^5$ is a repeating unit with a linear or branched alkylene group having 1 or more to 4 or less carbon atoms, $R^6$ is a hydrogen atom or a methyl group, $R^7$ is an alkyl group having 1 or more to 4 or less carbon atoms and a hydroxyl group bonded to at least one carbon atom, Y is $-COO^-$ or $-SO_3^-$, and m and n are each independently an integer of 1 or more)

Including the structural unit a2 improves film formability and adhesion to the base material. A proportion of the structural unit a2 in the polymer PA is, for example, 0 to 90 mol%.

In addition to the structural unit a1 represented by the above-mentioned formula (1), the polymer PA may include a repeating unit with the betaine structure other than the ester-bonded type (hereinafter referred to as a "structural unit a4"). Examples of the structural unit a4 include a repeating unit represented by the following formula (3).

[Chemical 3]

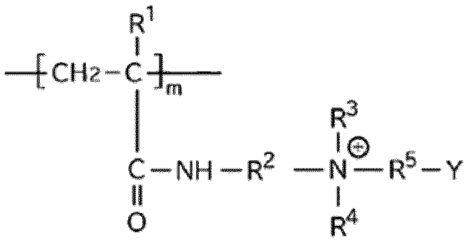

···(3)

a4

(In formula (3), R' is a hydrogen atom or a methyl group, $R^2$ is a linear or branched alkylene group having 1 or more to 6 or less carbon atoms, $R^3$ and $R^4$ are each independently an alkyl group having 1 or more to 4 or less carbon atoms, $R^5$ is a linear or branched alkylene group having 1 or more to 4 or less carbon atoms, Y is -COO- or $-SO_3^-$, and m is an integer of 1 or more)

A proportion of the structural unit a4 in the polymer PA is, for example, 0 to 90 mol%.

Examples of the repeating unit with the betaine structure other than the ester-bonded type described above include a repeating unit derived from a monomer with an amide-bonded betaine structure, such as 2-[dimethyl[3-(2-methylprop-2-enamide)-propyl]ammonio]acetate (MAMCMB), 3-[(3-methacryloylamino-propyl)-dimethyl-ammonio]-propionate (MAMCEB), 3-[(3-acryloylamino-propyl)-dimethyl-ammonio]propane-1-sulfonate (SPBAM), or 3-[(3-methacryloylamino-propyl)-dimethyl-ammonio]propane-1-sulfonate (SPBMAM).

That is, the polymer PA may be a homopolymer having the structural unit a1 represented by the above-mentioned formula (1) as a single repeating unit, or a copolymer having the structural unit a1 and the structural unit a2 represented by the above-mentioned formula (2). Further, the polymer PA may be a polymer having a structural unit based on other monomers. If the polymer is a copolymer, the polymer PA may be any of a random copolymer, a block copolymer, an alternating copolymer, and a graft copolymer of the structural unit a1 and the structural unit a2.

The above-mentioned polymer PA may further include at least one structural unit with a hydrophilic structure from the viewpoint of easily improving hydrophilicity and water swellability of the formed film. Examples of the hydrophilic structure in the structural unit with the hydrophilic structure include at least one structure selected from the group consisting of an amide structure (e.g., a (meth)acrylamide structure, etc.), an alkylene oxide structure, and a lactam structure (e.g., α-lactam (three-membered ring), β-lactam (four-membered ring), γ-lactam (five-membered ring), δ-lactam (six-membered ring), etc.). Specific examples of a monomer with the amide structure include (meth)acrylamide and N,N-dimethyl(meth)acrylamide. Specific examples of a monomer with the alkylene oxide structure include ethylene glycol and methoxy ethylene glycol. Specific examples of a monomer with the lactam structure include N-vinyl-2-caprolactam, N-vinyl pyrrolidone, and N-vinyl piperidone.

If the polymer PA further includes the structural unit with the hydrophilic structure, a proportion of the structural unit with the hydrophilic structure is preferably 20 mol% or more, more preferably 30 mol% or more, still more preferably 40 mol% or more, based on all structural units of the polymer PA from the viewpoint of easily improving hydrophilicity and water swellability of the formed film. Further, the proportion is preferably 60 mol% or less, more preferably 55 mol% or less, still more preferably 50 mol% or less, based on all structural units of the polymer PA from the viewpoint of easily improving water resistance and adhesion to the base material of the formed film.

In the coating agent CA of the present embodiment, the polymer PA is represented by, for example, the following formula (4).

[Chemical 4]

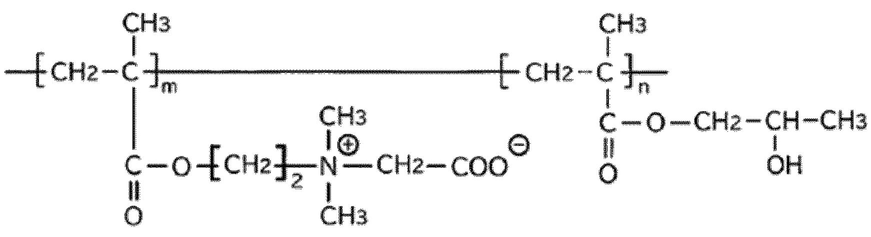

···(4)

CMB HPMA

(In formula (4), m and n are each independently an integer of 1 or more)

That is, in the present embodiment, the polymer PA is, for example, a random copolymer including a repeating unit derived from N-methacryloyloxyethyl-N,N-dimethylammonium-α-N-methylcarboxy betaine (CMB) as the structural unit a1, and a repeating unit derived from 2-hydroxypropyl methacrylate (HPMA) as the structural unit a2. In the present embodiment, a molar ratio of CMB relative to HPMA in the polymer PA is, for example, 90:10 to 10:90, preferably 60:40 to 40:60. In the present embodiment, the molecular weight of the polymer PA is, for example, 10,000 or more and 2,000,000 or less, and, for example, about 100,000.

Note that the polymer PA included in the coating agent CA of the present embodiment may include 2-hydroxyethyl methacrylate (HEMA) instead of HPMA in the above-mentioned formula (4), or may be a three-dimensional copolymer including HPMA and HEMA. Further, the polymer PA may include other repeating units in addition to the structural units a1 and a2. Examples of other repeating units include polyethylene glycol (PEG), methoxyethyl acrylate (MEA), n-butyl methacrylate (BMA), and 4-methacryloyloxybenzophenone (BEMA).

<Organic solvent HS>

Dissolving the polymer PA in the organic solvent HS can ensure the fluidity of the coating agent CA (specifically, the polymer PA) in a high-temperature environment. As a result, a swollen gel film GM described below can be favorably formed. Details will be described below.

The organic solvent HS of the present embodiment has a dispersion term δD of 10 to 24 $MPa^{1/2}$, a polar term δP of 5 to 19 $MPa^{1/2}$, and a hydrogen bond term δH of 3 to 17 $MPa^{1/2}$ in the Hansen solubility parameters at 25 °C. For example, if the hydrogen bond term δH of the Hansen solubility parameters exceeds 17 $MPa^{1/2}$, incompatibility of the solvent with the polymer PA becomes low, and, in some cases, the polymer PA becomes soluble, making it difficult to create a gel structure formed by the polymer PA. As a result, it becomes difficult to improve water resistance and swellability of the resulting film. Further, if the polymer PA is a polymer including the structural unit a1, the hydrolysis of the polymer PA described below is less likely to occur, and, as a result, a gel structure is hardly formed. If the hydrogen bond term δH is less than 3 and if the polar term δP is less than 5, the polymer PA no longer exhibits appropriate miscibility with water and may separate from water, making it difficult to create the gel structure from the polymer PA.

The above-mentioned dispersion term δD is 10 to 24 $MPa^{1/2}$, preferably 12 to 20 $MPa^{1/2}$, more preferably 15 to 19 $MPa^{1/2}$. The above-mentioned polar term δP is 5 to 19 $MPa^{1/2}$, preferably 8 to 17 $MPa^{1/2}$, more preferably 10 to 15 $MPa^{1/2}$. The above-mentioned hydrogen bond term δH is 3 to 17 $MPa^{1/2}$, preferably 5 to 14 $MPa^{1/2}$, more preferably 7 to 13 $MPa^{1/2}$.

As the Hansen solubility parameters of the organic solvent HS, values provided by the calculation software Hansen Solubility Parameter in Practice (HSPiP, developer; Charles M. Hansen) may be used. If the coating agent CA includes one type of organic solvent, it is sufficient that the Hansen solubility parameters of one organic solvent is within the above-mentioned range. If the coating agent CA includes two or more types of organic solvents, at least one of the organic solvents may satisfy the above-mentioned Hansen solubility parameters.

In the Hansen solubility parameters of the organic solvent HS, the dispersion term δD, the polar term δP, and the hydrogen bond term δH preferably satisfy the following relationship:

$$\sqrt{((\delta D-17)^2+(\delta P-12)^2+(\delta H-10)^2)}\leq 7$$

The value calculated by this formula is preferably 7 or less. The above-mentioned formula indicates that the organic solvent lies within a Hansen sphere having an interaction radius R of 7.0 with respect to a center value having the dispersion term δD of 17, the polar term δP of 12, and the hydrogen bond term δH of 10.

Further, the organic solvent HS of the present embodiment is a high boiling point organic solvent having the boiling point of higher than 100 °C, more preferably 110 °C or higher, still more preferably 115 °C or higher, even still more preferably 150 °C or higher, particularly preferably 180 °C or higher. The organic solvent HS of the present embodiment is more preferably a polar solvent, still more preferably a polar aprotic solvent. Further, from the viewpoint of manufacturability and availability of the resulting water-swellable film, the boiling point of the organic solvent HS is preferably 205 °C or lower. Note that, if the coating agent CA includes multiple types of organic solvents, it is sufficient that at least one type of the organic solvents has the above-mentioned boiling point.

The coating agent CA includes water in addition to the polymer PA and the organic solvent HS. In a configuration where the coating agent CA includes water, the coating agent CA includes a mixed solvent of water and the organic solvent HS, and the polymer PA, and the concentration of the polymer PA relative to the coating agent CA is, for example, preferably 1 wt% or more, more preferably 2 wt% or more, still more preferably 3 wt% or more. Further, the concentration is preferably 20 wt% or less, more preferably 15 wt% or less, still more preferably 10 wt% or less. Even still more preferably, the concentration may be about 5 wt%. Further, a proportion of the organic solvent HS in the above-mentioned

mixed solvent is, for example, preferably 5 wt% or more, more preferably 10 wt% or more, still more preferably 15 wt% or more. Further, the proportion is preferably 50 wt% or less, more preferably 45 wt% or less, still more preferably 40 wt% or less. Even still more preferably, the proportion is about 15 wt%.

A-2. Swollen gel film GM:

The swollen gel film GM of the present embodiment includes a polymer PB represented by the following general formula (5). More specifically, the swollen gel film GM is a hydrogel film swollen by including water, and a swelling degree of the swollen gel film GM is, for example, 180 % or more and 900 % or less, preferably 300 % or more and 800 % or less. Note that the above-mentioned swelling degree is calculated by d2/d1×100(%), in which d1 represents a film thickness when the swollen gel film GM is sufficiently dried (e.g., dried to a water content of 0.1% by weight or less), and d2 represents a film thickness when the swollen gel film GM is sufficiently swollen. Further, in the present embodiment, the swollen gel film GM means a physically crosslinked gel.

<Polymer PB>

As represented by the following formula (5), the polymer PB of the present embodiment preferably includes the above-mentioned structural unit a1 and a repeating unit derived from (meth)acrylic acid (hereinafter referred to as a "structural unit a3").

[Chemical 5]

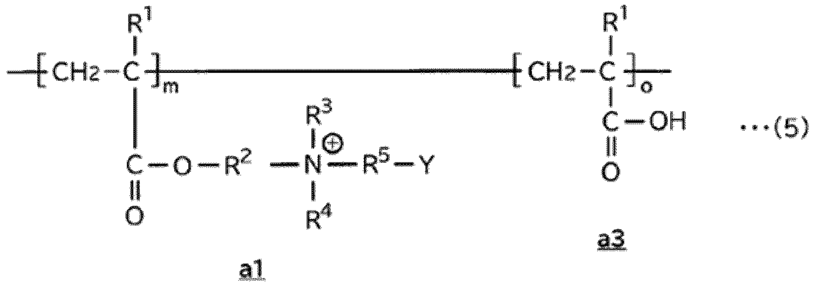

(In formula (5), R’ is a hydrogen atom or a methyl group, $R^2$ is a linear or branched alkylene group having 1 or more to 6 or less carbon atoms, $R^3$ and $R^4$ each independently an alkyl group having 1 or more to 4 or less carbon atoms, $R^5$ is a linear or branched alkylene group having 1 or more to 4 or less carbon atoms, Y is $-COO^-$ or $-SO_3^-$, and m and o are each independently an integer of 1 or more)

The polymer PB, as represented by the following general formula (6), may include the above-mentioned structural unit a2 in addition to the structural unit a1 and the structural unit a3 represented by the above-mentioned formula (5).

[Chemical 6]

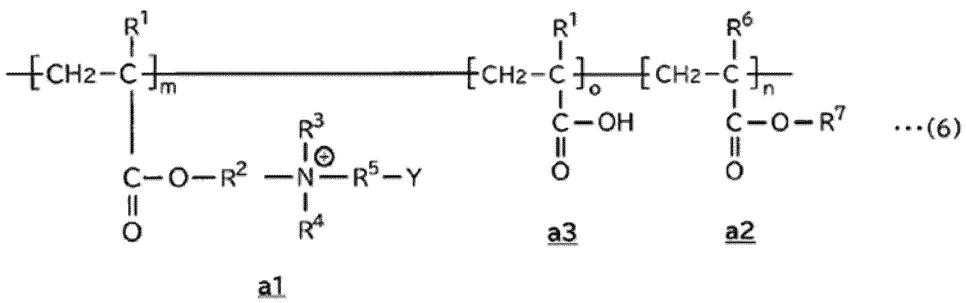

(In formula (6), R’ is a hydrogen atom or a methyl group, $R^2$ is a linear or branched alkylene group having 1 or more to 6 or less carbon atoms, $R^3$ and $R^4$ each independently an alkyl group having 1 or more to 4 or less carbon atoms, $R^5$ is a repeating unit with a linear or branched alkylene group having 1 or more to 4 or less carbon atoms, $R^6$ is a hydrogen atom or a methyl group, $R^7$ is an alkyl group having 1 or more to 4 or less carbon atoms and a hydroxyl group bonded

to at least one carbon atom, Y is $-COO^-$ or $-SO_3^-$, and m, n, and o are each independently an integer of 1 or more)

The above-mentioned polymer PB may not include the structural unit a1 as long as it includes the polymer unit with the betaine structure. For example, the polymer PB may be a polymer including the above-mentioned structural unit a4 and structural unit a3. The swollen gel film can be formed by interaction between the structural unit a4 and the structural unit a3. Such a polymer PB is obtained by using a polymer including the structural unit a1 and the structural unit a4 as the polymer PA, and hydrolyzing all the ester-bonded betaine structures of the structural unit a1, thereby creating the structural unit a3. Alternatively, the polymer PB may be a polymer including the structural unit a1, the structural unit a4, and the structural unit a3.

The polymer PB included in the above-mentioned swollen gel film includes the polymer unit with the betaine structure and the polymer unit with the carboxyl group. In the polymer PB, a ratio of the polymer unit with the betaine structure relative to the polymer unit with the carboxyl group is preferably 90:10 to 10:90 (molar ratio), more preferably 80:20 to 60:40 (molar ratio).

That is, the polymer PB may be a copolymer of the structural unit a1 and the structural unit a3, as represented by the above-mentioned formula (5), a copolymer of the structural unit a1, the structural unit a2, and the structural unit a3, as represented by the above-mentioned formula (6), or a polymer in which the structural unit a4 is further included in the structure represented by the above-mentioned formula (5) or formula (6). Alternatively, the polymer PB may be a copolymer of the structural unit a4 and the structural unit a3 or a copolymer of the structural unit a4, the structural unit a2, and the structural unit a3 without the structural unit a1. The polymer PB may be any of a random copolymer, a block copolymer, an alternating copolymer, and a graft copolymer including the above-mentioned structural units.

In the swollen gel film GM of the present embodiment, the polymer PB is represented by, for example, the following formula (7).

[Chemical 7]

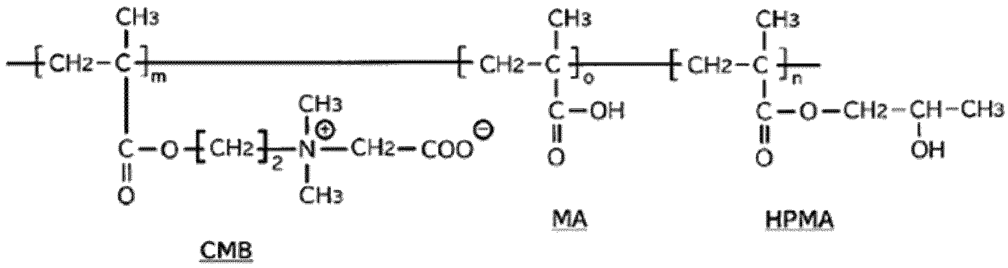

...(7)

(In formula (7), m, n and o are each independently an integer of 1 or more)

That is, in the present embodiment, the polymer PB is, for example, a random copolymer including a repeating unit derived from N-methacryloyloxyethyl-N,N-dimethylammonium-α-N-methylcarboxy betaine (CMB) as the structural unit a1, a repeating unit derived from 2-hydroxypropyl methacrylate (HPMA) as the structural unit a2, and a repeating unit derived from methacrylic acid (MA) as the structural unit a3. In the present embodiment, a molar ratio of CMB, HPMA, and MA in the polymer PB is, for example, 47:50:3 to 40:50:10, more preferably 45:50:5 to 43:50:7. In the present embodiment, the molecular weight of the polymer PB is, for example, 10,000 or more and 2,000,000 or less.

Note that the polymer PB included in the swollen gel film GM of the present embodiment may include 2-hydroxyethyl methacrylate (HEMA) instead of HPMA in the above-mentioned formula (7), or may be a three-dimensional copolymer including HPMA and HEMA. Further, the polymer PB may include other repeating units in addition to the structural units a1, a2, and a3. Examples of other repeating units include polyethylene glycol (PEG), methoxyethyl acrylate (MEA), n-butyl methacrylate (BMA), and 4-methacryloyloxybenzophenone (BEMA).

In the present embodiment, a proportion of the polymer PB in the swollen gel film GM is, for example, preferably 1 wt% or more and 30 wt% or less, more preferably 3 wt% or more and 20 wt% or less.

A-3. Guidewire 100:

Fig. 1 is an explanatory diagram schematically showing a configuration of a guidewire 100 of the present embodiment. Fig. 1 shows a configuration of a longitudinal section (YZ section) of the guidewire 100. Note that, in Fig. 1, illustration of a part of the guidewire 100 is omitted. In Fig. 1, the Z-axis positive direction side is the tip side (distal side) to be inserted into the body, and the Z-axis negative direction side is the base side (proximal side) to be operated by an operator such as a doctor. Although Fig. 1 shows a state in which the entire guidewire 100 has a straight shape substantially parallel to the Z-axis direction, the guidewire 100 is flexible enough to be bent.

Note that, in the present specification, for convenience of explanation, the guidewire 100 is assumed to be in

the state illustrated in Fig. 1, and the Z-axis direction is referred to as an "axial direction of the guidewire 100" or simply as an "axial direction", and the rotation direction around the Z-axis is referred to as a "circumferential direction of the guidewire 100" or simply as a "circumferential direction".

The guidewire 100 is an elongated medical device that is inserted into a blood vessel or the like in order to guide a catheter to a lesion (a site of stenosis or occlusion) in the blood vessel or the like. The total length of the guidewire 100 is, for example, about 1500 mm to 3000 mm, and the outer diameter of the guidewire 100 is, for example, about 0.5 to 1.2 mm.

The guidewire 100 includes a core shaft 10, a coil body 20, a tip-side joint portion 32, a base-side joint portion 34, and a resin portion 40 formed by the swollen gel film GM described above. At least one of the core shaft 10, the coil body 20, the tip-side joint portion 32, and the base-side joint portion 34 is an example of the base material in the claims.

The core shaft 10 is an elongated member having a small diameter at the tip side and a large diameter at the base side. More specifically, the core shaft 10 is configured by a rod-shaped small diameter portion 11, a rod-shaped large diameter portion 13 located on the base side relative to the small diameter portion 11 and having a larger diameter than the small diameter portion 11, and a tapered portion 12 located between the small diameter portion 11 and the large diameter portion 13 and having a diameter gradually increasing from a boundary position with the small diameter portion 11 to a boundary position with the large diameter portion 13. The shape of the cross section (XY cross section) at each position of the core shaft 10 can be any shape. For example, the cross section has a circular or flat plate shape. The outer diameter of the large diameter portion 13 is, for example, about 0.2 to 0.6 mm.

As a material constituting the core shaft 10, a known material is used. For example, a metal material, more specifically, stainless steel (SUS302, SUS304, SUS316, etc.), a Ni-Ti alloy, a piano wire, a nickel-chromium based alloy, a cobalt alloy, tungsten, or the like is used. The entire core shaft 10 may be constituted by the same material, or each part of the core shaft 10 may be constituted by different materials.

As shown in Fig. 1, the coil body 20 is a coil-shaped member formed into a hollow cylindrical shape by spirally winding a wire and is disposed so as to surround the outer periphery of the core shaft 10. As a forming material constituting the coil body 20, a known material is used. For example, a metal material, more specifically, stainless steel (SUS302, SUS304, SUS316, etc.), a Ni-Ti alloy, a piano wire, a nickel-chromium based alloy, a cobalt alloy, tungsten, or the like is used.

The tip-side joint portion 32 is a member that joins the tip end of the core shaft 10 and the tip end of the coil body 20. That is, the tip end of the core shaft 10 and the tip end of the coil body 20 are embedded and fixed inside the tip-side joint portion 32. The outer periphery surface of the tip-side joint portion 32 on the tip side is a smooth surface (e.g., a substantially hemispherical surface). Further, the base-side joint portion 34 is a member that joins the core shaft 10 and the base end of the coil body 20 at a predetermined position between the base end and the tip end of the core shaft 10 along the axial direction. That is, the base end of the coil body 20 is embedded and fixed inside the base-side joint portion 34.

As a material constituting the tip-side joint portion 32 and the base-side joint portion 34, a known material is used. For example, brazing metal (aluminum alloy brazing, silver brazing, gold brazing, etc.), metal solder (an Ag-Sn alloy, an Au-Sn alloy, etc.), an adhesive (an epoxy adhesive, etc.), or the like is used. In the present embodiment, brazing metal is used as the material for constituting the tip-side joint portion 32 and the base-side joint portion 34.

The resin portion 40 is a coating member that is formed of a resin and covers the outer periphery surfaces of the coil body 20, the tip-side joint portion 32, and the base-side joint portion 34. The resin portion 40 is constituted by the swollen gel film GM described above. The thickness of the resin portion 40 is, for example, about 0.01 to 0.1 mm. The resin portion 40 is disposed substantially uniformly on the outer periphery surface of the coil body 20 along the shape of the outer periphery surface of the wire.

A-4. Formation method of swollen gel film GM:

Next, an example of a method for forming the swollen gel film GM of the present embodiment will be described. Figs. 2A, B, C, and D are explanatory diagrams conceptually showing the method for forming the swollen gel film GM on a base material. Note that Figs. 2A, B, C, and D show a configuration of a part of the guidewire as the base material. Further, the method for forming the swollen gel film GM is part of the method for producing the guidewire 100.

First, a guidewire is prepared in which the core shaft 10, the coil body 20, the tip-side joint portion 32, and the base-side joint portion 34 are assembled (see Fig. 2A). Next, the coating agent CA described above is prepared. That is, the coating agent CA includes the polymer PA including the structural unit a1 which is a repeating unit with the ester-bonded betaine structure and the structural unit a2 which is a repeating unit derived from (meth)acrylic acid hydroxyalkyl ester, and the organic solvent HS. The coating agent CA is applied to the outer periphery surface of the coil body 20 and the outer surfaces of the tip-side joint portion 32 and the base-side joint portion 34 of the guidewire prepared above (hereinafter also referred to as a "guidewire surface") (application step, see Fig. 2B). A method for applying the coating agent CA is not particularly limited, and examples thereof include a dipping method (dip coating method) and a spraying

method.

Next, the coating agent CA is heated at a temperature of higher than 100 °C to form the swollen gel film GM (formation step, see Fig. 2C). More specifically, the swollen gel film GM is formed by hydrolysis of ester bonds in the polymer PA included in the coating agent CA. More specifically, for example, the guidewire coated with the coating agent CA is dried in a hot air circulation drying oven at a predetermined temperature for a predetermined period of time (e.g., 120 °C for 3 hours) to hydrolyze the ester bonds in the polymer PA. In the formation step, a hydrolysis rate of the ester bonds in the polymer PA is, for example, 20% or more and 40% or less, from the viewpoint of obtaining the swollen gel film GM that has both good water retention and good lubricity. In order to achieve the above-mentioned hydrolysis rate, the hydrolysis temperature in the formation step can be, for example, 110 °C or higher, more preferably 115 °C or higher and 135 °C or lower, and the hydrolysis time can be, for example, 30 minutes or more and 5 hours or less. In order to achieve the hydrolysis at such a high temperature, the organic solvent HS included in the coating agent CA can be selected according to the hydrolysis temperature. Through the above-mentioned steps, the swollen gel film GM (resin portion 40) can be formed on the surface of the guidewire (see Fig. 2D).

As described above, in the method for forming the swollen gel film GM of the present embodiment, using the coating agent CA makes it possible to form the swollen gel film GM by causing the hydrolysis to proceed only by heating without using a crosslinking agent that is generally used in a hydrolysis reaction. Below, a mechanism of forming the swollen gel film GM from the coating agent CA will be described. Note that, below, a configuration will be described in which the polymer PA included in the coating agent CA includes CMB as the structural unit a1 and HPMA as the structural unit a2 as represented by the above-mentioned formula (4). However, the following mechanism can be similarly applied to the polymer PA in which the structural unit a1 and the structural unit a2 are any of the above-mentioned repeating units, and the polymer PA that does not include the structural unit a2.

Fig. 3 shows the hydrolysis reaction in the formation step described above. In this reaction, through the formation step, a part of the repeating units of CMB constituting the polymer PA is hydrolyzed to produce the polymer PB including a repeating unit of MA. That is, hydrolyzing the polymer PA can produce the polymer PB including the repeating unit of CMB having a positive charge and the repeating unit of MA having a negative charge in one molecule. The carboxyl group in the repeating unit of MA described herein carries a high negative charge. Thus, it is speculated that a strong electrostatic interaction is favorably induced between the positively charged betaine structure and the highly negatively charged carboxyl group, and, as a result, gelation can proceed in each molecule in the polymer PB.

It is also speculated that the betaine structural part and the carboxyl group are, at least partially, converted to ampholytes by, for example, a reaction shown in the following drawing.

[Chemical 8]

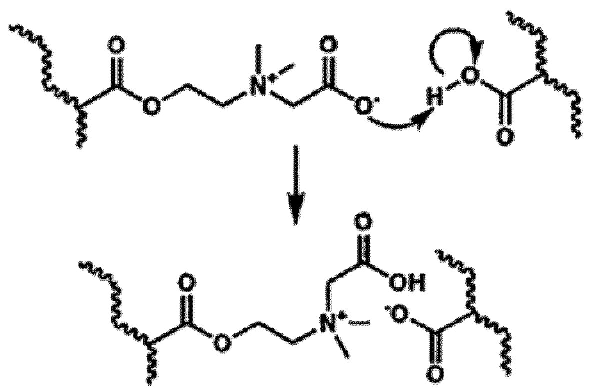

Thus, it is speculated that, when the polymer including the polymer unit with the ester-bonded betaine structure is heated at, for example, a temperature of higher than 100 °C, at which the ester-bonded part is hydrolyzed, in the presence of the specific organic solvent HS and water included in the coating agent CA, the following interaction occurs, and a crosslinked structure derived from the ampholytes is formed, so that a water-swellable film is formed.

[Chemical 9]

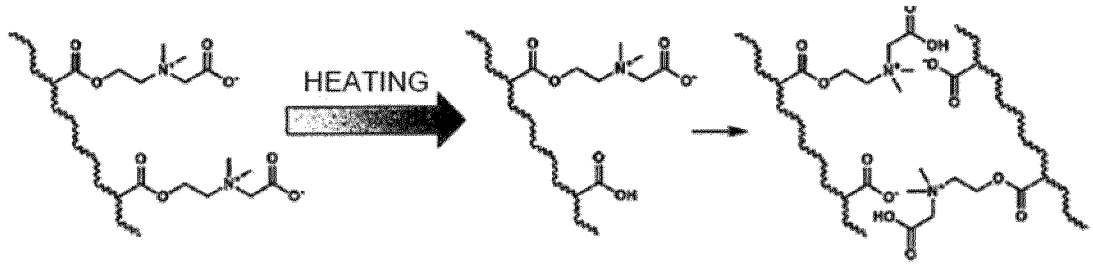


As described above, in the present embodiment, the coating agent CA includes the organic solvent HS. Thus, the swollen gel film GM can be favorably formed from the coating agent CA. The ester bonds in the polymer PA included in the coating agent CA are favorably hydrolyzed in a high temperature environment (e.g., 110 °C or higher). Thus, the coating agent CA including the organic solvent HS makes it possible to achieve a high-temperature environment where the ester bonds in the polymer PA can be favorably hydrolyzed while maintaining the fluidity of the coating agent CA (specifically, the polymer PA). As a result, the hydrolysis can proceed uniformly inside and outside the molecules of the polymer PA. Further, since the fluidity of the coating agent CA can be maintained in a high-temperature environment, the coating agent CA can ensure the flexibility of the molecular chains that constitute the polymer PB in the polymer PB produced through the hydrolysis. As a result, the betaine structure and the carboxyl group in the polymer PB can be brought close enough to induce a strong electrostatic interaction, allowing the gelation to proceed in the molecules. Thus, the coating agent CA including the organic solvent HS allows the hydrolysis of the polymer PA to proceed uniformly, and as a result, makes it possible to form the swollen gel film GM with good lubricity.

A-5. Performance evaluation:

The performance of the guidewire 100 including the swollen gel film GM (resin portion 40) was evaluated for the following items. First, a method for producing samples S1 to S5 will be described.

<Sample S1>

(Synthesis of polymer PA)

First, 0.014 mol of CMB (manufactured by Osaka Organic Chemical Industry Ltd., trade name: GLBT) and 0.014 mol of HPMA (manufactured by FUJIFILM Wako Pure Chemical Corp., trade name: Hydroxypropyl Methacrylate) were added to and dissolved in 20 g of a mixed solution (water:ethanol ratio of 50:50). While stirring the resulting solution, 0.00026 mol of ammonium persulfate (manufactured by Tokyo Chemical Industry Co., Ltd., trade name: APS) was further added to and dissolved in the solution. The resulting solution was stirred and reacted in a constant temperature bath at 60 °C for 20 hours to obtain a polymer solution including a CMB-HPMA copolymer. The resulting polymer solution was added dropwise to a 20-fold volume of an acetone solution to be recrystallized, followed by filtration and vacuum drying to obtain a polymer solid (polymer PA). The number average molecular weight of the obtained polymer PA was approximately 100,000. The number average molecular weight of the polymer PA was measured using NMR (nuclear magnetic resonance) and GPC (gel permeation chromatography).

(Preparation of coating agent CA)

The polymer PA was dissolved in a mixed solution including the organic solvent HS (manufactured by FUJIFILM Wako Pure Chemical Corp., trade name: 1-Methyl-2-pyrrolidone (NMP)) (distilled water:NMP ratio of 70:30) to prepare a 5 wt% polymer solution. In this performance evaluation, in order to increase viscosity of the polymer solution, 0.3 wt% of carboxymethyl cellulose (CMC, manufactured by Sigma-Aldrich, trade name: CMC Ultra High Viscosity) was added as a thickener to the above-mentioned polymer solution, and the resulting mixture was stirred to prepare the coating agent CA. Note that the added amount of CMC can be adjusted as appropriate depending on the thickness of the swollen gel film GM to be formed on the base material. More specifically, CMC may not be added, or a low viscosity agent may be added instead of CMC. Further, other thickeners may be added instead of CMC.

(Formation method of swollen gel film GM)

The coating agent CA described above was applied to the guidewire including the core shaft 10, the coil body

20, the tip-side joint portion 32, and the base-side joint portion 34 by a dip coating method. After that, the hydrolysis was carried out at a heating temperature of 120 °C for a heating time of 3 hours. Specifically, the coating agent CA was dried for 3 hours in a hot air circulation drying oven at 120 °C to form the swollen gel film GM (resin portion 40) on the guidewire, thereby producing a sample S1.

A decomposition rate of the polymer was calculated by quantifying HCMB [2-(2-hydroxyethyldimethylammonio)acetate] produced by the hydrolysis of the ester-bonded betaine structure using liquid chromatography, and applying the amount of HCMB (mol) and the theoretical amount of GLBT (mol, the charged amount) in the polymer to the following formula.

Decomposion rate (%) = HCMB (mol)/theoretical GLBT (mol) in polymer × 100

<Samples S2 to S5>

Samples S2 to S5 were produced under the same conditions as the sample S1, except that the preparation conditions were changed as shown in Table 1 below.

<Performance evaluation results>

Table 1 below shows evaluation results for lubricity and film strength of the samples S1 to S5. For the lubricity, the samples S1 to S5 were each immersed in physiological saline, and then evaluation was made based on the feel when the swollen gel film GM part was pinched and rubbed between fingertips. In this feel evaluation, a slippery feel was evaluated as lubricity "A" (lubricity: high), and a rough feel was evaluated as lubricity "B" (lubricity: low). Further, when the presence of the swollen gel film GM was not confirmed, the lubricity was evaluated as "C" (no swollen gel film). Further, the film strength was evaluated by measuring a resistance value. That is, the lower the resistance value, the higher the film strength. For measuring the resistance value, each of the samples S1 to S5 was sandwiched between an upper urethane roller (manufactured by Misumi Corp., AXFM-D25-L15-V8-N) and a lower stainless steel plate (SUS304, 30 mm x 30 mm), and the weight of the urethane roller was adjusted to 100 g. Under such conditions, the sample was pulled while water was running around it, and a resistance load of the sample was measured with a force gauge. Further, the initial resistance value and the 50th resistance value were measured, and durability was evaluated by comparing the two values. The initial resistance value is a resistance value obtained in the first measurement, and the 50th resistance value is a resistance value obtained in the fiftieth measurement after the same measurement was continuously repeated fifty times.

[Table 1]

| | Preparation condition | | | Evaluation results | | | | |
|---|---|---|---|---|---|---|---|---|
| | NMP | Drying temperature (°C) | Drying time (h) | Lubricity evaluation | Initial resistance values (gf) | 50th resistance values (gf) | Decomposition rate | Swelling degree |
| Sample S1 | Yes | 120 | 3 | A | 5 | 7 | 35 | 728 |
| Sample S2 | Yes | 130 | 1 | A | 4 | 7 | 20 | 500 |
| Sample S3 | Yes | 140 | 1 | A | 16 | 18 | 31 | 186 |
| Sample S4 | Yes | 80 | 3 | B | 30 | 60 | 0 | 114 |
| Sample S5 | No | 120 | 3 | C | 10 | 80 | 14 | 127 |

In the lubricity evaluation results, the samples S1 to S3 were evaluated as "A" (lubricity: high), while the sample S4 was evaluated as "B" (lubricity: low). As described above, in the samples S1 to S3, the heating temperature is 120 °C, while, in the sample S4, the heating temperature is as low as 80°C. Thus, it was speculated that, in the sample S4,

this was caused by the low heating temperature at the time of drying the swollen gel film GM, resulting in the insufficient hydrolysis of the polymer PA included in the coating agent CA. That is, it was speculated that, compared to the samples S1 to S3 in which the polymer PA was sufficiently hydrolyzed, the sample S4 lacked the repeating unit (structural unit a3) derived from MA in the polymer PB after the hydrolysis, resulting in the low electrostatic interaction with the betaine structure in the polymer PB and the insufficient progress of the gelation.

The lubricity evaluation result of the sample S5 was "C" (no swollen gel film). That is, in the sample S5, the presence of the swollen gel film on the guidewire was not confirmed. This was thought to be due to the fact that, in the sample S5, the coating agent not including the organic solvent HS failed to achieve a high-temperature environment in which the ester bonds in the polymer PA could be favorably hydrolyzed. That is, it was speculated that gelation of the polymer PB did not proceed, and the coating agent was washed away when immersed in the above-mentioned physiological saline.

In the film strength evaluation results, the samples S1 to S3 in which the swollen gel films GM were favorably formed had the lower initial resistance values and lower 50th resistance values than the samples S4 and S5. That is, this could confirm that the samples S1 to S3 had the higher film strength than the samples S4 and S5. Further, in the samples S1 to S3, the 50th resistance values equivalent to the initial resistance values could be obtained. In other words, this means that the swollen gel films GM in the samples S1 to S3 have the good film strength and durability. This could confirm that the samples S1 to S3 had the good film strength and durability while having the good lubricity.

A-6. Effects of the present embodiment:

As described above, the coating agent CA of the present embodiment includes the polymer PA including the polymer unit (structural unit a1) with the ester-bonded betaine structure, and the organic solvent HS, as the organic solvent, having the dispersion term δD of 10 to 24 $MPa^{1/2}$, the polar term δP of 5 to 19 $MPa^{1/2}$, and the hydrogen bond term δH of 3 to 17 $MPa^{1/2}$ in the Hansen solubility parameters at 25 °C, and having the boiling point of higher than 100 °C. Since the coating agent CA of the present embodiment includes the organic solvent HS, the swollen gel film GM can be favorably formed from the coating agent CA. More specifically, the ester bonds in the polymer PA included in the coating agent CA are favorably hydrolyzed in a high temperature environment (e.g., 110 °C or higher). Thus, the coating agent CA of the present embodiment including the organic solvent HS can achieve a high-temperature environment in which the ester bonds in the polymer PA can be favorably hydrolyzed while maintaining the fluidity of the coating agent CA (specifically, polymer PA). As a result, the hydrolysis can proceed uniformly inside and outside the molecules of the polymer PA. Further, since the fluidity of the coating agent CA can be maintained in a high-temperature environment, the coating agent CA of the present embodiment can ensure the flexibility of the molecular chains constituting the polymer PB in the polymer PB produced through the hydrolysis. The polymer PB described herein includes the polymer unit (structural unit a1) having the ester-bonded betaine structure with a positive charge and the (meth)acrylic acid polymer unit (structural unit a3) with a negative charge in one molecule. Further, the carboxyl group included in the (meth)acrylic acid polymer unit (structural unit a3) is highly negatively charged. As described above, in the coating agent CA of the present embodiment, the flexibility of the molecular chains constituting the polymer PB can be ensured, thus, the positively charged betaine structure and the highly negatively charged carboxyl structure can be brought close enough to induce a strong electrostatic interaction in the polymer PB. This allows the gelation to proceed in the molecules of the polymer PB. As a result, according to the coating agent CA of the present embodiment including the organic solvent HS, the hydrolysis of the polymer PA can proceed uniformly without going through complicated steps, in other words, simply by heating at a temperature of higher than 100 °C, which, in turns, makes it possible to form the swollen gel film GM with good lubricity. Further, according to the coating agent CA of the present embodiment, it is possible to apply the coating agent CA to the surface of the base material (such as the coil body 20 of the guidewire 100) with a relatively low viscosity, making it possible to form the swollen gel film GM on the surface of a medical instrument with a complex surface shape such as the guidewire 100 while minimizing detachment of the swollen gel film GM from the surface.

In the coating agent CA of the present embodiment, the organic solvent HS is an organic solvent having the dispersion term δD of 10 to 24 $MPa^{1/2}$, the polar term δP of 5 to 19 $MPa^{1/2}$, and the hydrogen bond term δH of 3 to 17 $MPa^{1/2}$ in the Hansen solubility parameters at 25 °C, and having the boiling point of higher than 100 °C. In particular, the organic solvent HS is at least one selected from N-methyl-2-pyrrolidone (NMP), dimethyl sulfoxide (DMSO), N,N-dimethylformamide (DMF), dimethylacetamide (DMA), diacetone alcohol (DAOH), and diethylene glycol mono ethyl ether (EDG). Using the above-mentioned aprotic polar solvent with a relatively high boiling point (e.g., 150 °C or higher) as the above-mentioned organic solvent HS can favorably maintain the fluidity of the coating agent CA in the high-temperature environment described above. Thus, according to the coating agent CA of the present embodiment, the hydrolysis of the polymer PA can proceed more effectively and uniformly, which, in turns, makes it possible to form the swollen gel film GM with good lubricity.

The swollen gel film GM of the present embodiment includes the polymer PB including the polymer unit (structural unit a1) with the ester-bonded betaine structure and the (meth)acrylic acid polymer unit (structural unit a2). That is,

according to the swollen gel film GM of the present embodiment, the polymer PB included in the swollen gel film GM includes the polymer unit (structural unit a1) having the ester-bonded betaine structure with a positive charge and the negatively charged (meth)acrylic acid polymer unit (structural unit a3) in one molecule. Further, the carboxyl group included in the (meth)acrylic acid polymer unit (structural unit a3) is highly negatively charged. Thus, in the swollen gel film GM of the present embodiment, a strong electrostatic interaction is favorably induced between the positively charged betaine structure and the highly negatively charged carboxyl group in each molecule constituting the polymer PB. As a result, it becomes possible to favorably maintain the gelation state in each molecule.

The guidewire 100 of the present embodiment is coated with the swollen gel film GM described above. Thus, according to the guidewire 100 of the present embodiment, it is possible to provide the guidewire 100 including the swollen gel film GM in which the gelation state is favorably maintained in each molecule.

The method for forming the swollen gel film GM of the present embodiment includes the application step of applying the coating agent CA onto the base material and the formation step of forming the swollen gel film GM by heating the coating agent CA at a temperature of higher than 100 °C. The coating agent CA includes the polymer PA including the polymer unit (structural unit a1) with the ester-bonded betaine structure, and the organic solvent HS having the boiling point of higher than 100 °C. In the formation step, the swollen gel film GM is formed by hydrolyzing the ester bonds in the polymer PA. According to the method for forming the swollen gel film GM of the present embodiment, the coating agent CA includes the polymer PA including the polymer unit (structural unit a1) with the ester-bonded betaine structure, and the organic solvent HS having the boiling point of higher than 100 °C. Thus, the swollen gel film GM can be favorably formed on the base material without going through complicated steps.

In the method for forming the swollen gel film GM of the present embodiment, the base material is a guidewire. Further, in the formation step, the hydrolysis rate of the ester bonds in the polymer PB is 20% or more and 40% or less. According to the method for forming the swollen gel film GM of the present embodiment, the swelling degree can be appropriately adjusted, making it possible to form the swollen gel film GM having lubricity suitable for the guidewire 100.

[Reference examples (resin compositions 1 to 16)]

A polymer solution was obtained in the same manner as the synthesis of the polymer PA, except that GLBT, HPMA, and MAA were dissolved in water at a molar ratio of 43:50:7 and a total monomer concentration of 10%. The viscosity average molecular weight of the obtained polymer was 100,000. The polymer solution thus obtained and NMP were mixed at a mass ratio of 1:0.15 to obtain a resin composition. The mass ratio of the copolymer, the organic solvent, and water in the resin composition was as follows: copolymer:organic solvent:water=8.7:13.0:78.3

Resin compositions 5-2 to 5-16 were obtained in the same manner as the synthesis of the polymer PA, except that, in the resin composition including the copolymer obtained above (indicated as "resin composition 5-1" in Table 2), each of the solvents shown in Table 2 was used instead of NMP. Insoluble film formability, water resistance, and water swellability of these resin compositions were measured in the same manner as above. The results are shown in Table 2.

(Insoluble film formability)

After each resin composition was spread on a Teflon (registered trademark) coated tray, the resin composition was dried at 85 °C for 3 hours using a commercially available hot air dryer. Ninety parts of water was added to 10 parts of the obtained copolymer solid. After being stirred at room temperature for 30 minutes, the mixture was allowed to stand for 24 hours, and a state of the solution was evaluated according to the following criteria.

A: Clear solution

B: Solution with undissolved residues or cloudy solution

(Water resistance visual evaluation)

After 2.5 parts of each resin composition was spread on a Teflon (registered trademark) coated tray (10 cm x 10 cm), the resin composition was allowed to stand in an atmosphere at 125 °C for 3 hours to obtain a cured product. The cured product was peeled off from the tray, 2 parts of the peeled cured product was placed in a container, 98 parts of water was added to the container, and the mixture was allowed to stand at room temperature for 24 hours. Then, a state of the solution was visually examined and evaluated according to the following criteria. Note that evaluation results shown in parentheses indicate predicted values. The same applies to other evaluations described below.

A: Cured product remaining in swollen state

B: Cured product being dissolved or remaining in unswollen state

(Water swellability visual evaluation)

Two parts of the cured product obtained in the same manner as described above for the water resistance was placed in a container, 98 parts of water was added to the container, and the mixture was allowed to stand at room temperature for 24 hours. Then, a state of the solution was visually examined and evaluated according to the following criteria.

A: Cured product remaining in swollen state

B: Cured product being dissolved or remaining in unswollen state

[Table 2]

| Resin compositions | Organic solvents | | | | | Resin composition evaluation results | | |
|---|---|---|---|---|---|---|---|---|
| | Type | Hansen solubility parameters | | | Boiling point (°C) | Insoluble film formability (85 °C) | Water resistance | Water swellability |
| | | δD | δP | δH | | | | |
| 5-1 | NMP | 18.0 | 12.3 | 7.2 | 202.0 | A | A | A |
| 5-2 | Acetone | 15.5 | 10.4 | 7.0 | 56.5 | B | B | B |
| 5-3 | Acetonitrile | 15.3 | 18.0 | 6.1 | 82.0 | B | B | B |
| 5-4 | n-Butyl acetate | 15.8 | 3.7 | 6.3 | 126.0 | B | B | B |
| 5-5 | Diacetone alcohol | 15.8 | 8.2 | 10.8 | 166.0 | A | A | A |
| 5-6 | Diethylene glycol | 16.6 | 12.0 | 19.0 | 245.0 | B | B | B |
| 5-7 | Diethylene glycol monoethyl ether | 16.1 | 9.2 | 12.2 | 196.0 | A | A | A |
| 5-8 | Dimethylacetamide | 16.8 | 11.5 | 9.4 | 165.0 | A | A | A |
| 5-9 | Dimethylformamide | 17.4 | 13.7 | 11.3 | 153.0 | A | A | A |
| 5-10 | Dimethyl sulfoxide | 18.4 | 16.4 | 10.2 | 189.0 | A | A | A |
| 5-11 | Ethanol | 15.8 | 8.8 | 19.4 | 78.3 | B | B | B |
| 5-12 | Ethylene glycol | 17.0 | 11.0 | 26.0 | 197.0 | B | B | B |
| 5-13 | Methanol | 14.7 | 12.3 | 22.3 | 64.7 | B | B | B |
| 5-14 | Tetrahydrofuran | 16.8 | 5.7 | 8.0 | 66.0 | B | B | B |
| 5-15 | Toluene | 18.0 | 1.4 | 2.0 | 110.6 | B | B | B |
| 5-16 | Water | 15.5 | 16.0 | 42.3 | 100.0 | B | B | B |

B. Modifications:

In the above-mentioned embodiment, the base material coated with the swollen gel film GM may be a medical instrument other than the guidewire 100, or other base materials that require good lubricity.

Although the above-mentioned embodiment has been described using the guidewire as an example of the elongated medical instrument, the technology disclosed herein is applicable to other elongated medical instruments such as a catheter in the same manner. Below, the elongated medical instrument will be described.

Elongated medical instrument:

The elongated medical instrument of the present disclosure can be an elongated medical instrument which is

used by being inserted into the body. Specifically, the elongated medical instrument of the present disclosure can be an elongated medical instrument in which, for example, an elongated medical instrument whose outer periphery is made of metal or an elongated medical instrument whose outer periphery is formed of a resin such as urethane is used as a base material, and the previously mentioned swollen gel film is formed on the surface of the base material. Particularly preferable examples of the elongated medical instrument of the present disclosure include a guidewire and a catheter. Specifically, for example, a guidewire whose outer periphery is made of metal, a catheter with a hollow shaft whose outer periphery is made of a resin such as polyurethane, or the like can be used as the base material.

The catheter of the present disclosure is not particularly limited, and it can be applicable to any catheter such as, for example, a guiding catheter, a penetrating catheter, a microcatheter, a balloon catheter, a foreign body removal catheter, a contrast catheter, a bile duct catheter, a urinary catheter, an endoscope, or a dilator. Further, the guidewire of the present disclosure is not particularly limited, and it can be applicable to any guidewire such as, for example, a PCI guidewire for percutaneous coronary treatment, a PTA guidewire for lower extremity vascular treatment, an IVR guidewire for peripheral vascular treatment, an INR guidewire for cerebrovascular treatment, or a CAG guidewire for contrast imaging.

More specifically, the elongated medical instrument of the present embodiment can adopt various configurations, for example, as shown in (a) to (e) below. The swollen gel films in the following (a) to (e) each includes the polymer (a1) including the polymer unit with the betaine structure and the polymer unit with the carboxyl group described herein. Further, as a material for forming the following coating layer and tubular member, a resin is preferable, and examples of the resin include polyamide, polyimide, modified polyolefin, polyvinyl alcohol, polyurethane, polyurea, polyester, polyether, polylactic acid, and a combination thereof.

(a) A guidewire including a linear core wire, a coating layer provided on at least a part of the outer periphery of the core wire, and a swollen gel film formed on the surface of the coating layer.

(b) A guidewire including a linear core wire, a coil layer in which a wire is spirally wound around at least a part of the outer periphery of the core wire, and a swollen gel film formed on the surface of the coil layer.

(c) A guidewire including a linear core wire, a coil layer in which a wire is spirally wound around at least a part of the outer periphery of the core wire, a coating layer provided on the outer periphery of the coil layer, and a gel film formed on the surface of the coating layer.

(d) A catheter including a tubular member and a swollen gel film formed on the surface of the tubular member.

(e) A catheter including a tubular member, a balloon disposed at one end of the tubular member, and a swollen gel film formed on the surface of the balloon.

Note that the elongated medical instrument of the present embodiment may have a different configuration from the above (a) to (e), or may be an elongated medical instrument other than the guidewire and the catheter. It is sufficient that at least a part of the surface of the elongated medical instrument is provided with the swollen gel film described above.

DESCRIPTION OF REFERENCE NUMERALS

10: Core shaft

11: Small diameter portion

12: Tapered portion

13: Large diameter portion

20: Coil body

32: Tip-side joint portion

34: Base-side joint portion

40: Resin Portion

100: Guidewire

CA: Coating agent

GM: Swollen gel film

HS: Organic solvent

PA: Polymer

PB: Polymer

## Claims

**1.** An elongated medical instrument comprising:

a base material; and
a swollen gel film coating the base material, wherein the swollen gel film includes a polymer (a1) including a repeating unit with a betaine structure and a repeating unit with a carboxyl group (excluding the polymer unit with the betaine structure).

**2.** The elongated medical instrument according to claim 1, wherein the repeating unit with the betaine structure includes a repeating unit with an ester-bonded betaine structure.

**3.** The elongated medical instrument according to claim 1 or 2, wherein the swollen gel film has a swelling degree of 180 % or more and 900 % or less.

**4.** The elongated medical instrument according to any one of claims 1 to 3, wherein the swollen gel film has a molar ratio of the polymer unit with the betaine structure relative to the polymer unit with the carboxyl group in the polymer (a1) of 80:20 to 60:40.

**5.** The elongated medical instrument according to any one of claims 1 to 4, wherein the base material is a guidewire or a catheter.

**6.** A method for producing an elongated medical instrument, comprising;

an application step of applying a coating agent onto a base material; and
a formation step of forming a swollen gel film by heating the coating agent, wherein:
the coating agent includes:

a polymer having a repeating unit with an ester-bonded betaine structure;
an organic solvent having a dispersion term $\delta D$ of 10 to 24 $MPa^{1/2}$, a polar term $\delta P$ of 5 to 19 $MPa^{1/2}$, and a hydrogen bond term $\delta H$ of 3 to 17 $MPa^{1/2}$ in Hansen solubility parameters at 25 °C, and having a boiling point of higher than 100 °C; and
water; and
in the formation step, the swollen gel film is formed by hydrolyzing an ester bond in the polymer.

**7.** The method for producing the elongated medical instrument according to claim 6, wherein the organic solvent is at least one selected from N-methyl-2-pyrrolidone (NMP), dimethyl sulfoxide (DMSO), N,N-dimethylformamide (DMF), dimethylacetamide (DMA), diacetone alcohol (DAOH), and diethylene glycol monoethyl ether (EDG).

**8.** The method for producing the elongated medical instrument according to claim 6 or 7, wherein:

the base material is a guidewire or a catheter; and
in the formation step, a hydrolysis rate of the ester bond in the polymer is 20% or more and 40% or less.

FIG.1

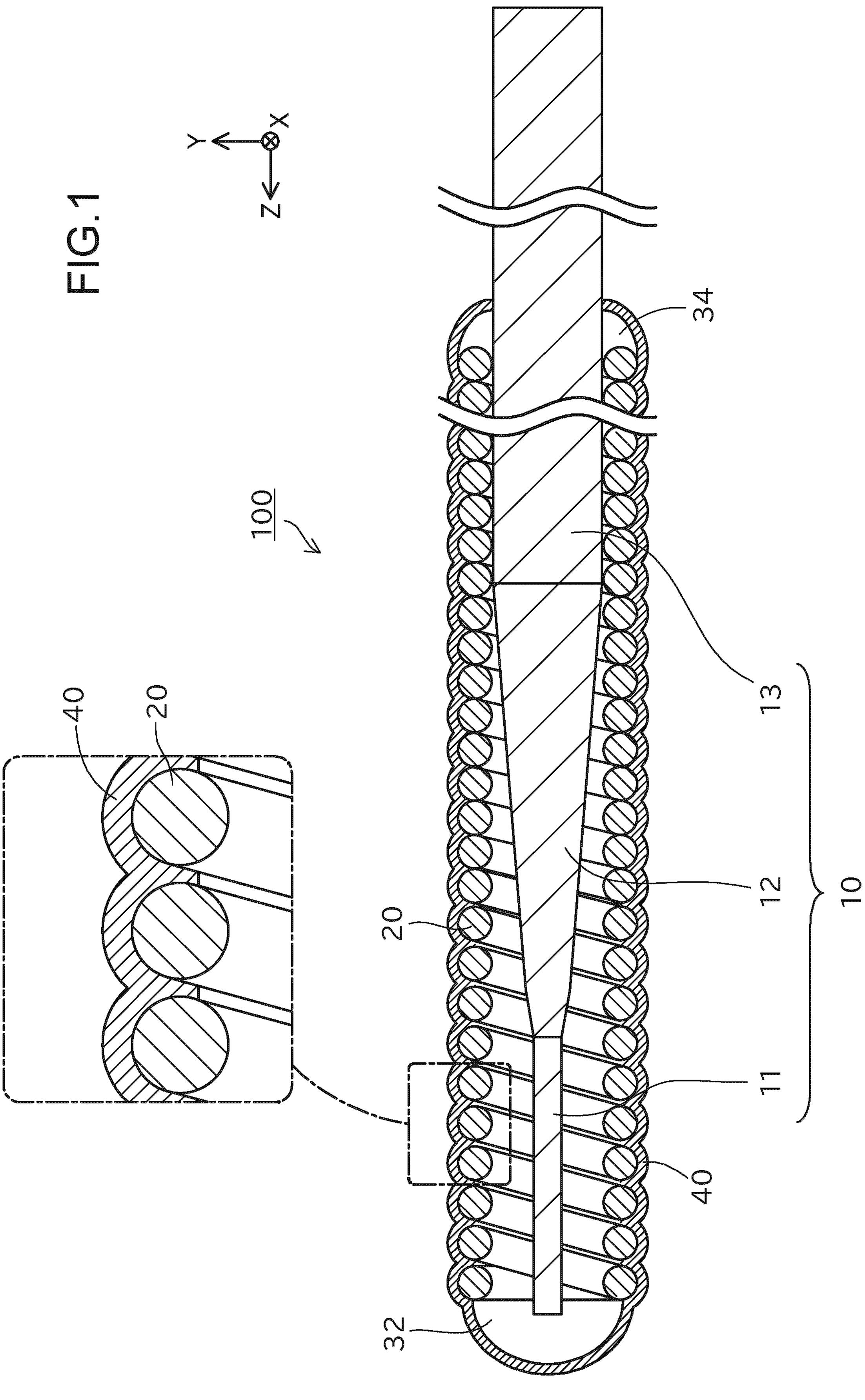

FIG.2

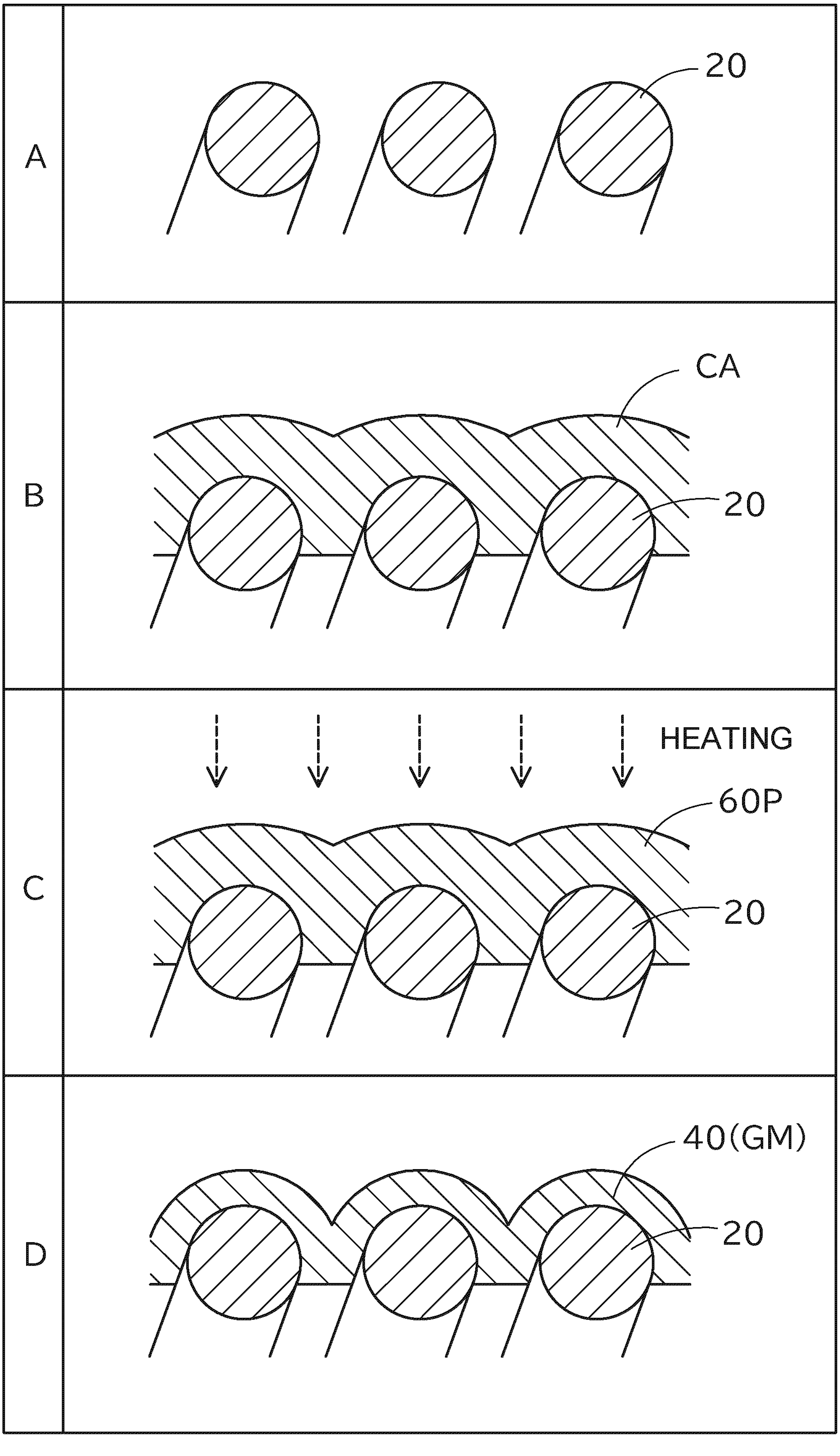

FIG.3

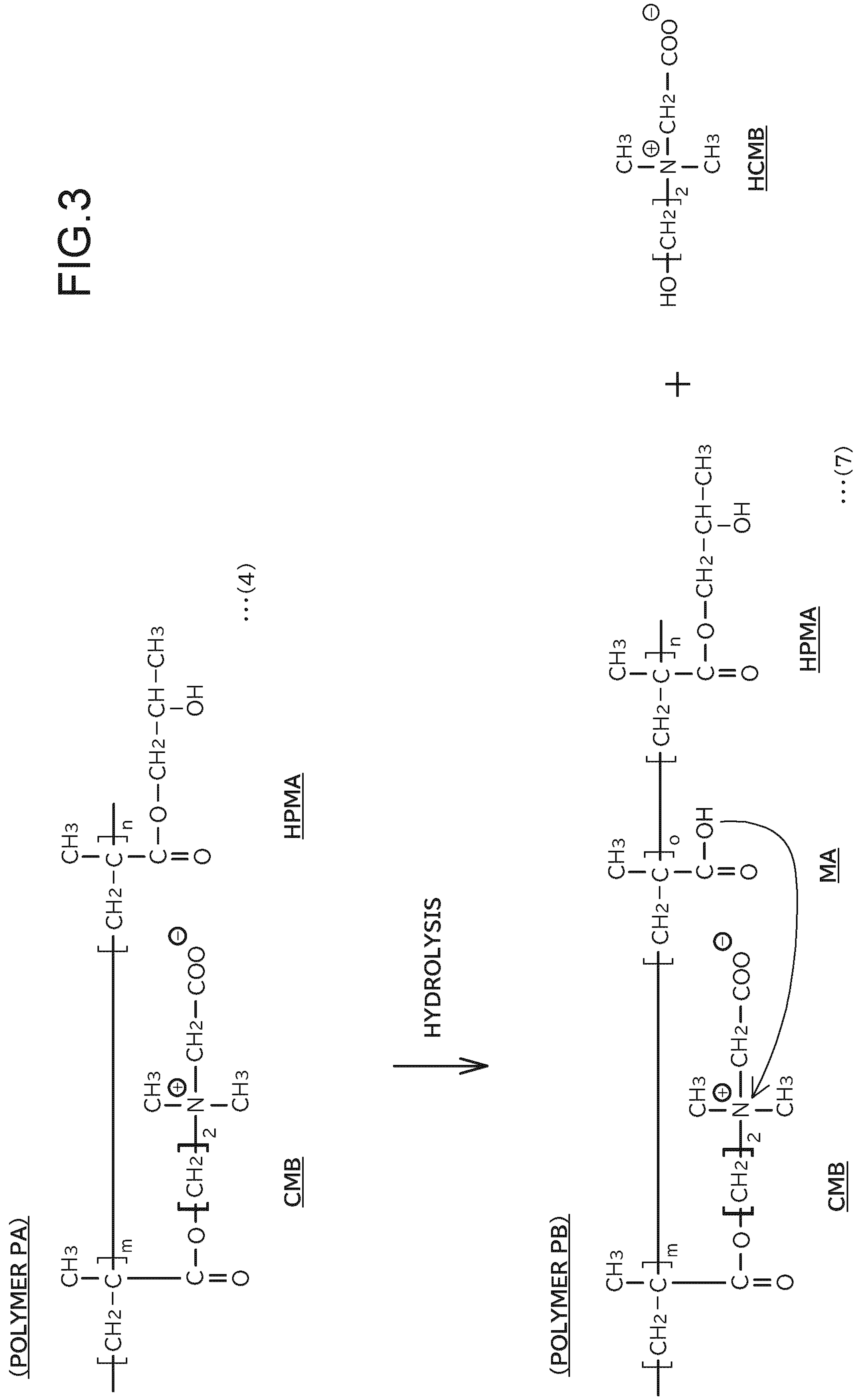


(POLYMER PA)

CMB

HPMA

…(4)

(POLYMER PB)

CMB

MA

HPMA

…(7)

HCMB

**INTERNATIONAL SEARCH REPORT**

International application No.
**PCT/JP2022/039207**

**A. CLASSIFICATION OF SUBJECT MATTER**

***A61L 29/08***(2006.01)i; ***A61L 27/16***(2006.01)i; ***A61L 27/52***(2006.01)i; ***A61L 29/14***(2006.01)i; ***A61M 25/00***(2006.01)i; ***A61M 25/09***(2006.01)i
FI: A61L29/08 100; A61M25/09 516; A61M25/00 610; A61M25/00 500; A61M25/09 550; A61L29/14 300; A61L27/16; A61L27/52

According to International Patent Classification (IPC) or to both national classification and IPC

**B. FIELDS SEARCHED**

Minimum documentation searched (classification system followed by classification symbols)

A61L29/08; A61L27/16; A61L27/52; A61L29/14; A61M25/00; A61M25/09

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2007-130194 A (OSAKA ORGANIC CHEM. IND. LIMITED) 31 May 2007 (2007-05-31)<br>claims, examples 1-5 | 1-8 |
| A | WO 2016/052618 A1 (ASAHI KASEI MEDICAL COMPANY, LIMITED) 07 April 2016 (2016-04-07)<br>claims, examples 1-11 | 1-8 |
| A | US 2016/0355624 A1 (SHENZHEN UNIVERSITY) 08 December 2016 (2016-12-08)<br>claims, examples 1-5 | 1-8 |
| A | JP 2013-237737 A (HOKKAIDO UNIVERSITY) 28 November 2013 (2013-11-28)<br>claims, examples 1-3 | 1-8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 January 2023** | **24 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/039207**

| Patent document cited in search report | Publication date (day/month/year) | Patent family member(s) | Publication date (day/month/year) |
|---|---|---|---|
| JP 2007-130194 A | 31 May 2007 | (Family: none) | |
| WO 2016/052618 A1 | 07 April 2016 | US 2017/0216504 A1<br>claims, examples 1-11<br>EP 3202432 A1<br>CN 106794290 A | |
| US 2016/0355624 A1 | 08 December 2016 | WO 2016/011729 A1<br>CN 104193893 A | |
| JP 2013-237737 A | 28 November 2013 | US 2015/0133566 A1<br>claims, examples 1-3<br>WO 2013/172292 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018524029 W